# EUROPEAN PATENT SPECIFICATION

(11) **EP 1 556 505 B1**
(45) Date of publication and mention of the grant of the patent: **27.05.2009**
(21) Application number: 03773650.1
(22) Date of filing: 17.10.2003
(51) Int. Cl.: C12Q 1/48

(54) **MODULATION OF S6 KINASE ACTIVITY FOR THE TREATMENT OF OBESTIY**
MODULATION DER S6-KINASEAKTIVITÄT ZUR BEHANDLUNG VON ADIPOSITAS
MODULATION DE L'ACTIVITE DE LA KINASE S6 POUR LE TRAITEMENT DE L'OBESITE

(30) Priority: 18.10.2002 GB 0224338; 22.08.2003 US 497227 P
(43) Date of publication of application: 27.07.2005
(73) Proprietor: Novartis Forschungsstiftung, Zweigniederlassung, 4058 Basel (CH)
(72) Inventor: FRIGERIO, Francesca, CH-4058 Basel (CH); FUMAGALLI, Stefano, CH-4058 Basel (CH); KOZMA, Sara, C., F-68220 Hésingue (FR); STICKER-JANTSCHEFF, Melanie, 79211 Denzlingen (DE); THOMAS, George, F-68220 Hésingue (FR); UM, Sung, Hee, CH-4058 Basel (CH)
(74) Representative: Ott, Johann
(86) International application number: PCT/EP2003/011554
(87) International publication number: WO 2004/035815

(56) References cited:
- WO-A-00/08173
- WO-A-00/66721
- WO-A-01/44497
- WO-A-97/34137
- PHAM PHUONG-TRUC T ET AL: "Assessment of cell-signaling pathways in the regulation of mammalian target of rapamycin (mTOR) by amino acids in rat adipocytes" JOURNAL OF CELLULAR BIOCHEMISTRY, vol. 79, no. 3, 7 September 2000 (2000-09-07), pages 427-441, XP002269150 ISSN: 0730-2312
- FASSHAUER MATHIAS ET AL: "Hormonal regulation of adiponectin gene expression in 3T3-L1 adipocytes" BIOCHEMICAL AND BIOPHYSICAL RESEARCH COMMUNICATIONS, vol. 290, no. 3, 25 January 2002 (2002-01-25), pages 1084-1089, XP002269151 ISSN: 0006-291X

### SEQUENCE LISTING

<110> Novartis Forschungsstiftung, Zweigniederlassung Friedrich Miescher Institute for Biomedical Research
<120> Modulation of S6 kinase activity for the treatment of obesity
<130> 1-32727A/FMI
<160> 4
<170> PatentIn version 3.1
<210> 1
   <211> 19
   <212> DNA
   <213> Artificial Sequence
<220>
   <223> Description of Artificial sequence:oligonucleotide (RNA or DNA)
<400> 1
   agtgtttgac atagacctg 19
<210> 2
   <211> 21
   <212> DNA
   <213> Artificial sequence
<220>
   <223> Description of Artificial sequence:oligonucleotide (RNA or DNA)
<400> 2
   aagggggcta tggaaaggtt t 21
<210> 3
   <211> 10
   <212> PRT
   <213> homo sapiens
<400> 3
   Lys Arg Arg Arg Leu Ala Ser Leu Ala Ala
   1               5                   10
<210> 4
   <211> 20
   <212> PRT
   <213> homo sapiens
<400> 4
   Lys Arg Arg Arg Leu Ser Ser Leu Arg Ala Ser Thr Ser Lys Ser Glu
   1               5                   10                  15
   Ser Ser Gln Lys
   20

## Description

The current invention relates to obesity, in particular the treatment of obesity with modulators of S6 kinase (S6K) activity.

### BACKGROUND OF THE INVENTION

Obesity is a metabolic disease frequently associated with insulin resistance and together constitute risk factors for later development of type II diabetes and cardiovascular diseases. Insulin resistance occurs well before development of type II diabetes, and insulin is overproduced to compensate for the insulin resistance and to maintain normal glucose levels. Insulin resistance can later develop into type II diabetes, as the pancreas can no longer produce enough insulin to maintain normal glucose levels. Early stages of type II diabetes are associated with elevated levels of insulin but as the disease progresses the levels of insulin but as the disease progresses the pancreas may fail to produce insulin, resulting in increased blood glucose levels. Diabetes is a significant risk factor for both heart disease and stroke and is the leading cause of blindness and end-stage renal failure.

There are over 1 billion overweight individuals worldwide with 100 million clinically obese. The increasing health care costs of treating obesity related diseases in the US alone are estimated at over $100 billion annually. Current methods for treating obesity include behavioural modification, dieting, surgery (gastroplasty), administering pharmaceutical agents that block appetite stimulating signals or absorption of nutrients (fat), and administering agents that increase thermogenesis or fat metabolism. Some of these methods have disadvantages in that they rely on patient resolve, are invasive, or have unwanted side effects. An understanding of the mechanisms by which obesity is regulated may provide important therapeutic information.

Intracellular signalling pathways have been implicated in various cellular functions and the involvement of kinases in such pathways provides potential drugable targets for modulating a signalling pathway. Much work has been carried out in attempting to delineate the S6 kinase (S6K) signalling pathway. S6 kinase is a kinase that phosphorylates the ribosomal protein, S6. Deletion of the S6K1 gene (also known as p70/p85 S6K) in mice led to the identification of an S6K1 homologue, S6K2, which can partly compensate for S6K1 function biochemically to phosphorylate S6 (Shima et al., 1998, EMBO J., 17, 6649-6659). The precise function of S6K2 in vivo is not known to date.

S6K1-deficient mice are viable and fertile but exhibit a conspicuous reduction in body size during embryogenesis, an effect mostly overcome by adulthood. A comparison of homozygous mutant mice at 3.5 weeks of age demonstrated that the weights of all organs were proportional to the reduction in body weight. The small size of the homozygous mutant mice is consistent with a defect in translational capacity (Shima et al., 1998, EMBO J., 17, 6649-6659).

Mice deficient for S6K1 are hypoinsulinaemic and mildly glucose intolerant, not due to a lesion in glucose sensing or insulin production, but to a reduction in pancreatic endocrine mass, which is accounted by a selective decrease in beta-cell size. Thus, hypoinsulinaemia and not insulin resistance is thought to be the primary lesion predisposing S6K1-deficient mice to hyperglycaemia. The observed phenotype closely parallels the form of preclinical type 2 diabetes mellitus, where malnutrition-induced hypoinsulinaemia predisposes individuals to glucose intolerance (Pende et al., 2000, Nature, 408, 994-997).

S6K activity has been implicated in cancer and angiogenesis. WO93/19752 describes the use of rapamycin and its derivatives as inhibitors of p70 S6 kinase and their use to inhibit proliferation or the immune response of a cell. US 2003/0083284 describes antisense compounds for the inhibition of expression of p70 S6 kinase (referring to both the 70kDa and nuclear, 85kDa isoforms). The antisense nucleic acids are proposed to be potentially useful in treating infections, inflammation and tumor formation, as well as metabolic disorders. US2003/0143656 proposes that compounds capable of increasing the activity of p70 S6K may be useful in treating diabetes or obesity, or may be useful in inhibiting apoptosis.

Attoub et al. (2001, Faseb J., 14, 2329-2338) show that rapamycin blocks leptin function, in particular leptin-induced invasion of cells into collagen gels (as a model of carcinogenesis). Leptin therapy has been used to promote weight loss while preserving lean mass in obese patients with congenital leptin deficiency, suggesting leptin in the treatment of obesity or diabetes.

There remains a need for non-invasive therapies to promote weight loss in obese individuals with improved specificity to avoid side-effects and the present invention meets this need.

### SUMMARY OF THE INVENTION

In accordance with a first aspect of the invention, a method of identifying an agent effective in treating weight disorders dependent on fat accumulation is provided, the method comprising the steps of: i) incubating S6 kinase with a compound; ii) detecting S6 kinase activity; and iii) determining a compound-induced modulation in the S6 kinase activity relative to when the compound is absent, wherein an alteration of the S6 kinase activity in the presence of the compound is indicative of an agent effective in treating weight disorders dependent on fat accumulation. The compound -induced modulation is preferably independent of an effect of mammalian Target of Rapamycin (mTOR) activity. In one embodiment, the modulation is inhibition of S6 kinase 1 activity and the weight disorder is obesity or an overweight condition. In an alternative embodiment, the modulation is activation of S6 kinase 1 activity and the weight disorder is an underweight condition resulting from insufficient fat accumulation. S6 kinase activity can be conveniently assayed using S6 or a peptide as a substrate and is easily amenable to high throughput assays.

Also provided by the invention are methods of screening for an agent effective in treating weight disorders, comprising contacting transcriptionally active cellular components with a nucleic acid encoding an S6K gene operably linked to a promoter sequence or an S6K promoter sequence operably linked to a reporter gene in the presence of at least one compound; and detecting an effect of the compound on S6 kinase expression or S6 kinase promoter activity, wherein detection of a decrease or an increase in S6 kinase expression or promoter activity is indicative of an agent effective in treating weight disorders dependent on fat accumulation. Such assays can be cell-based assays, where the transcriptionally active cellular components and nucleic acid is present in a cell. In preferred embodiments, the S6 kinase is S6 kinase 1.

The screening assays of the invention preferably include the step of detecting an effect on fat accumulation, fat metabolism or adipocyte size by the compound.

Also encompassed are agents identified by the methods of the invention.

In a further aspect, a method for reducing adipocyte size is provided, comprising contacting an adipocyte with an effective amount of an S6 kinase 1 inhibitor (an inhibitor that preferentially reduces S6 kinase 1 activity compared to S6 kinase 2).

In yet another aspect, methods for treating a weight disorder dependent on fat accumulation, comprising administering to a subject a pharmaceutically effective amount of an S6 kinase modulator are provided. The S6 modulator can be an S6K1 inhibitor, in particular where the weight disorder is obesity or an overweight condition and may include inhibitory antibodies or fragments thereof specific for S6 kinase 1 (e.g., preferentially reducing catalytic activity of S6K1 compared to S6K2) or an antisense, ribozyme or siRNA that preferentially reduces expression of S6 kinase 1 compared to S6 kinase 2.

Thus, also provided are the use of specific modulators of S6 kinase for the manufacture of a medicament for the treatment or prophylactic treatment of a weight disorder dependent on fat accumulation, such as a selective inhibitor of S6 kinase 1 (e.g., an antisense molecule, a ribozyme, an siRNA, an antibody or fragment thereof) for the treatment or prophylactic treatment of an overweight condition, such as obesity.

Also provided by the invention is an at least partially double stranded RNA comprising a nucleic acid sequence of AGUGUUUGACAUAGACCUG (SEQ ID NO:1) or AAGGGGGCUAUGGAAAGGUUU (SEQ ID NO:2) for use as a medicament.

In a further aspect of the invention, a method of diagnosing a predisposition to a weight disorder dependent on fat accumulation is provided, comprising: obtaining a sample from an individual, detecting the level of S6 kinase activity, preferably S6 kinase 1 activity, in the sample and correlating a change in S6 kinase activity in the sample when compared to a normal control value or range of values with a predisposition to a weight disorder dependent on fat accumulation. For example, an increase in S6 kinase 1 activity when compared to a normal control value or range of values is indicative of a predisposition to obesity.

### DETAILED DESCRIPTION OF THE INVENTION

The present invention is based upon the finding that S6K1 deficient mice maintain their lower body weight relative to wild type mice as they age, even when placed on a high fat diet. S6K1 deficient mice eat the same total amount of food, but compared to body weight, they eat much more. Dissection of S6K1 deficient mice reveals a severe reduction of white fat and brown fat (e.g., in epidedymal fat), whereas organ size is unaffected. The reduction in fat is due to a reduction in fat cell (adipocyte) size. The S6K1-/- mice are protected against fat accumulation, due to a sharp increase in basal lipolysis and a highly elevated metabolic rate. Thus, it is expected that modulation of S6K1 activity will be useful in treating weight disorders. Although mTOR (mammalian target of rapamycin, which phosphorylates and activates S6 kinase) can be targeted directly to modulate S6K1 activity, direct targeting of S6K1 avoids more general side-effects of inhibiting mTOR activity, thereby providing more specificity for the treatment of patients with weight disorders. In particular, mTOR is known to activate both S6K1 and S6K2, whereas the present inventors have found that the selective inhibition of S6K1 is desirable.

Accordingly, the present invention provides a method of identifying an agent effective in treating weight disorders dependent on fat accumulation, such as obesity, based on the modulation of S6 kinase activity, in particular S6 kinase 1 activity. Typically such a method will comprise the steps of incubating S6 kinase (or a functional equivalent or derivative thereof) with a compound; detecting S6 kinase activity; and determining the compound-induced modulation in the S6 kinase activity relative to when the compound is absent. An alteration of the S6 kinase activity in the presence of the compound is indicative of an agent effective in treating weight disorders. A control assay in the absence of the compound can be run in parallel.

Unless otherwise clear from the context, "S6K" or "S6 kinase" is used herein to encompass both S6K1 (p70 and p85) and S6K2 (see, for example, Genebank Accession No. M57428, AJ007938, AB019245, NM003952 and related sequences), although S6K1 is a preferred target. Exemplary functional equivalents (variants) or derivatives of S6K include molecules where S6K is covalently modified by substitution, chemical, enzymatic, or other appropriate means with a moiety other than a naturally occurring amino acid.

Generally speaking such variants will be substantially homologous to the 'wild type' or other sequence specified herein i.e. will share sequence similarity or identity therewith. Similarity or identity may be at the nucleotide sequence and/or encoded amino acid sequence level, and will preferably, be at least about 50%, 60%, or 70%, or 80%, most preferably at least about 90%, 95%, 96%, 97%, 98% or 99%. Sequence comparisons may be made using FASTA and FASTP (see Pearson & Lipman, 1988. Methods in Enzymology 183: 63-98). Parameters are preferably set, using the default matrix, as follows: Gapopen (penalty for the first residue in a gap): -12 for proteins / -16 for DNA; Gapext (penalty for additional residues in a gap): -2 for proteins / -4 for DNA; KTUP word length: 2 for proteins /6 for DNA. Analysis for similarity can also be carried out using hybridisation. One common formula for calculating the stringency conditions required to achieve hybridization between nucleic acid molecules of a specified sequence homology is: Tm = 81.5oC + 16.6Log [Na+] + 0.41 (% G+C) - 0.63 (% formamide) - 600/#bp in duplex (Molecular Cloning: a Laboratory Manual: 2nd edition, Sambrook et al., 1989, Cold Spring Harbor Laboratory Press).

Variants that retain common structural features can be fragments of S6K, in particular fragments maintaining catalytic activity or isoform specific characteristics. For example, the carboxy- terminal sequences of S6K1 and S6K2 exhibit only about 20% identity and it therefore may be useful to include such isoform-specific features in fragments when isoform-specific assays are desired. Similarly, S6K1 can be phosphorylated at T447, which is absent in S6K2 providing an alternative or additional isoform-specific feature. Preferably, fragments will be between 50 and 350 amino acids in length.

Variants of S6K also comprise mutants thereof, which may contain amino acid deletions, additions or substitutions, subject to the requirement to maintain at least one feature characteristic of S6K, preferably catalytic activity and/or isoform-specific features as described above. Thus, conservative amino acid substitutions may be made substantially without altering the nature of S6K, as may truncations. Additions and substitutions may moreover be made to the fragments of S6K used in the screening methods of the invention, in particular those enhancing S6K catalytic activity or providing some other desirable property. For example, T389, T229 and S371 in mouse S6K1 (also known as p70S6K) are homologous to T389, T238 and S380 in Drosophila p70S6K. T389 is particularly indicated for mutation to an acidic amino acid residue in order to produce a constitutively active kinase. Fusion proteins with the S6K or S6K fragments referred to above may also be desirable.

The screening assays of the invention are not limited to any particular method of determining S6 kinase activity. S6 kinase assays are well known in the art (see for example USPN 6,372,467, which is herein incorporated by reference in its entirety). Briefly, S6 kinase will be incubated with a suitable substrate, such as S6, in a buffer allowing phosphorylation of S6. Phosphorylation of the substrate can be detected using a labelled phosphate group, such as the use of the radioactive label ³²P present as the ATP source in the buffer. Alternatively, antibodies specific for the phosphorylated products of S6K catalytic activity can be used to detect activity. As will be apparent to those of ordinary skill in the art, the assays are easily amenable to high through-put technologies using robotics and automated processes.

Alternatively, the S6 kinase activity can be assayed using a synthetic substrate, such as those comprising Arg- (Arg)-Arg-X-X-Ser-X (e.g., KRRRLASLAA, SEQ ID NO:3; or KRRRLSSLRASTSKSESSQK, SEQ ID NO:4) (Flowtow and Thomas (1992) J. Biol. Chem. 267: 3074-3078.

S6K activity can also be assayed by detecting downstream targets of the kinase. For example, S6K is known to affect transcription and translation of specific targets, such as genes with polypyrimidine tracts (5'TOPs) and ribosomal genes. (Fumagalli S, Thomas G. (1999) Ribosmal Protein S6 Phosphorylation and Signal Transduction. In: Translational Control. Eds. Hershey, J, Mathews, M, Sonenberg, N. Cold Spring Harbor Press. pp 695-717).

Thus, in accordance with a further aspect of the invention, a method is provided for screening an agent effective in treating a weight disorders dependent on fat accumulation (e.g. obesity), by identifying compounds that modulate expression of an S6K gene or a gene expressed under the control of S6K regulatory sequences.

The methods comprise contacting transcriptionally active cellular components, preferably in a cell, with a nucleic acid encoding an S6K gene operably linked to a promoter sequence or an S6K promoter sequence (or other S6K regulatory regions allowing expression of the reporter gene) operably linked to a reporter gene in the presence of at least one compound; and detecting an effect of the compound on expression of the coding region, be it S6 kinase expression or reporter gene expression. A decrease or an increase in S6 kinase expression or promoter activity is indicative of an agent effective in treating weight disorders. Such assays can be cell-based assays, where the transcriptionally active cellular components and nucleic acid is present in a cell, although in vitro transcription assays are also well known in the art. In preferred embodiments, the S6 kinase is S6 kinase 1.

The reporter gene encodes any molecule capable of providing a detectable change. Such reporter molecules include fluorescent moieties (e.g., fluorescent proteins, such as, cyan fluorescent protein, CFP; yellow fluorescent protein, YFP; blue fluorescent protein, BFP; or green fluorescent protein, GFP; all available commercially, Clontech Living Colors User Manual, antigens, reporter enzymes and the like. Reporter enzymes include, but are not limited to, the following: beta-galactosidase, glucosidases, chloramphenicol acetyltransferase (CAT), glucoronidases, luciferase, peroxidases, phosphatases, oxidoreductases, dehydrogenases, transferases, isomerases, kinases, reductases, deaminases, catalases and urease. In selecting a reporter molecule to be used in the presently claimed method, the reporter molecule itself should not be inactivated by any putative agent or other component present in the screening assay, including inactivation by any protease activity present in the assay mixture. The selection of an appropriate reporter molecule will be readily apparent to those skilled in the art.

The nucleic acid will typically be provided in a vector allowing replication in one or more selected host cells, as is well known for a variety of bacteria, yeast, and mammalian cells. For example, various viral origins (SV40, polyoma, adenovirus, VSV or BPV) are useful for cloning vectors in mammalian cells. The vector may, for example, be in the form of a plasmid, cosmid, viral particle, phage, or any other suitable vector or construct which can be taken up by a cell and used to express the sequence of interest or reporter gene.

Expression vectors usually contain a promoter operably linked to the protein-encoding nucleic acid sequence of interest, so as to direct mRNA synthesis. Promoters recognized by a variety of potential host cells are well known, as are the S6K1 and S6K2 promoters (upstream regulatory sequences). "Operably linked" means joined as part of the same nucleic acid molecule, suitably positioned and oriented for transcription to be initiated from the promoter. DNA operably linked to a promoter is "under transcriptional control" of the promoter. Transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g. the actin promoter or an immunoglobulin promoter, and from heat-shock promoters, provided such promoters are compatible with the host cell systems. Expression vectors of the invention may also contain one or more selection genes, such as genes conferring resistance to antibiotics or other toxins.

The methods of the invention may therefore further include introducing the nucleic acid into a host cell. The introduction, which may (particularly for in vitro introduction) be generally referred to without limitation as "transformation", may employ any available technique. For eukaryotic cells, suitable techniques may include calcium phosphate transfection, DEAE-Dextran, electroporation, liposome-mediated transfection and transduction using retrovirus or other virus, e.g. vaccini, as is well known in the art. See, for example, Keown et al., Methods in Enzymology, 185:527 537 (1990) and Mansour et al., Nature 336:348-352 (1988).

Host cells transfected or transformed with expression or cloning vectors described herein may be cultured in conventional nutrient media. The culture conditions, such as media, temperature, pH and the like, can be selected by the skilled artisan without undue experimentation. In general, principles, protocols, and practical techniques for maximizing the productivity of cell cultures can be found in "Mammalian Cell Biotechnology: a Practical Approach", M. Butler, ed. JRL Press, (1991) and Sambrook et al, supra.

Assays specific for S6K1 can also be designed by detecting binding of specific proteins, such as nerabin to the C-terminal domain of S6K1, as is well known in the art (Burnett PE, Blackshaw S, Lai MM, Qureshi IA, Burnett AF, Sabatini DM, Snyder SH. Neurabin is a synaptic protein linking p70 S6 kinase and the neuronal cytoskeleton. Proc Natl Acad Sci U S A. 1998 Jul 7;95(14):8351-6).

Thus, in another embodiment of the invention the inhibition of the interaction of S6K1 with a binding partner is assessed. This may comprise (i) contacting S6K1 with a binding partner thereof in the presence and absence of a test substance; and (ii) determining whether the presence of a test substance inhibits the interaction between S6K1 and its binding partner.

Methods for assessing the interaction between a polypeptide and a binding partner may be any of the methods known to those skilled in the art and are disclosed here. Any of these methods can be used to assess whether a test substance inhibits the interaction between a polypeptide (in this case S6K1) and a binding partner.

In one embodiment, assays are those based upon S6K1 and its interaction with neurabin and comprise the step of determining whether the test substance inhibits the interaction between S6K1 and neurabin. This may be achieved by detecting the physical association between S6K1 and its binding partner through labelling one with a detectable label and bringing it into contact with the other which has been immobilised on a solid support. Suitable detectable labels include ³⁵S-methionine which may be incorporated into recombinantly produced S6K1 and/or the binding partner thereof. The recombinantly produced S6K1 and/or binding partner may also be expressed as a fusion protein containing an epitope which can be labelled with an antibody. Alternatively, double-labelling may be used as is well known in the art, for example, using a radioactive label and a scintillant.

Generally, a protein which is immobilized on a solid support may be immobilized using an antibody against that protein bound to a solid support or via other technologies which are known per se. A preferred in vitro interaction may utilise a fusion protein including a tag, such as glutathione-S-transferase (GST) or His6. The tag may be immobilized by affinity interaction, for example on glutathione agarose beads or Ni-matrices, respectively.

In an in vitro assay format of the type described above the putative inhibitor compound can be assayed by determining its ability to modulate the amount of labelled S6K1 or binding partner which binds to the immobilized binding partner, e.g., GST-binding partner or GST-S6K1 as the case may be. This may be determined by fractionating the glutathione-agarose beads by SDS-polyacrylamide gel electrophoresis. Alternatively, the beads may be rinsed to remove unbound protein and the amount of protein which has bound can be determined by counting the amount of label present in, for example, a suitable scintillation counter.

Alternatively an antibody attached to a solid support and directed against one of S6K1 or the binding partner may be used in place of GST to attach the molecule to the solid support. Antibodies against S6K1 and its binding partners may be obtained in a variety of ways known as such in the art. In an alternative mode, one of S6K1 and its binding partner may be labelled with a fluorescent donor moiety and the other labelled with an acceptor, which is capable of reducing the emission from the donor. This allows an assay according to the invention to be conducted by fluorescence resonance energy transfer (FRET). In this mode, the fluorescence signal of the donor will be altered when S6K1 and its binding partner interact. The presence to a candidate inhibitor that modulates the interaction will increase the amount of fluorescence signal of the donor.

FRET is a technique known per se in the art and thus the precise donor and acceptor molecules and the means by which they are linked to S6K1 and its binding partner may be accomplished by reference to the literature.

Suitable fluorescent donor moieties are those capable of transferring fluorogenic energy to another fluorogenic molecule or part of a compound and include, but are not limited to, coumarins and related dyes such as fluoresceins, rhodols and rhodamines, resorufins, cyanine dyes, bimanes, acridines, isoindoles, dansyl dyes, aminophthalic hydrazines such as luminol and isoluminol derivatives, aminophthalimides, aminonaphthalimides, aminobenzofurans, aminoquinolines, dicyanohydroquinones, and europium and terbium complexes and related compounds.

Suitable acceptors include, but are not limited to, coumarins and related fluorophores, xanthenes such as fluoresceins, rhodols and rhodamines, resorufins, cyanines, difluoroboradiazaindacenes, and phthalocyanines.

A preferred donor is fluorescein and preferred acceptors include rhodamine and carbocyanine. The isothiocyanate derivatives of these fluorescein and rhodamine, available from Aldrich Chemical Company Ltd, Gillingham, Dorset, UK, may be used to label S6K1 and its binding partner. For attachment of carbocyanine, see for example Guo et al, J. Biol. Chem., 270; 27562-8, 1995.

Assays of the invention may also be performed in vivo. Such an assay may be performed in any suitable host cell, e.g. a bacterial, yeast, insect or mammalian host cell. Yeast and mammalian host cells are particularly suitable. To perform such an assay in vivo, constructs capable of expressing S6K1 and its binding partner and a reporter gene construct may be introduced into the cells. This may be accomplished by any suitable technique, for example calcium phosphate precipitation or electroporation. The constructs may be expressed transiently or as stable episomes, or integrated into the genome of the host cell.

In vivo assays may also take the form of two-hybrid assays. Two-hybrid assays may be in accordance with those disclosed by Fields and Song, 1989, Nature 340; 245-246. In such an assay the DNA binding domain (DBD) and the transcriptional activation domain (TAD) of the yeast GAL4 transcription factor are fused to the first and second molecules respectively whose interaction is to be investigated. A functional GAL4 transcription factor is restored only when two molecules of interest interact. Thus, interaction of the molecules may be measured by the use of a reporter gene operably linked to a GAL4 DNA binding site, which is capable of activating transcription of said reporter gene. Other transcriptional activator domains may be used in place of the GAL4 TAD, for example the viral VP16 activation domain.

Irrespective of the form of assay used, they will typically be run with suitable controls routine to those of skill in the art and is preferably used to screen compounds that may be present in small molecule libraries, peptide libraries, phage display libraries or natural product libraries. Putative or actual inhibitors or other modulators may be provided from any source which it is desired to screen, and may or may not be naturally occurring or synthetic, and may or may not be peptides or polypeptides (e.g. antibodies) or nucleic acids (e.g. siRNA). Preferred inhibitors most suited for therapeutic applications will be small molecules e.g. from a combinatorial library such as are now well known in the art (see e.g. Newton (1997) Expert Opinion Therapeutic Patents, 7(10): 1183-1194). Preferred candidate substances may include small molecules such as PKC inhibitors.

A compound-induced modulation of S6K activity means that there is a change in S6K activity (enzymatic activity, signalling activity to downstream targets, promoter activity or expression) in the presence of the compound relative to when the compound is absent. In particular a compound induced inhibition of S6K activity is reflected by a decrease in S6K activity relative to when the compound is absent. Conversely, a compound induced activation of S6 activity is reflected by an increase in S6K activity.

Activators and inhibitors are referred to collectively herein as modulators and preferably influence the kinase activity of S6K directly. Assays carried out using reconstituted components can be easily designed to achieve direct S6K inhibition (i.e., specific inhibition of S6K catalytic activity and not inhibition of the formation of active kinase, for example, through the action of mTOR). Typically, S6 kinase 1 activity will be selectively inhibited (i.e., preferentially over S6 kinase 2 activity and other kinases and enzymes.), in particular when inhibition of S6 kinase 1 signalling in adipose tissue and weight loss is desired. Conversely, S6K1 activity may be activated or S6K2 activity inhibited when the weight disorder is an underweight condition resulting from insufficient fat accumulation.

S6K inhibitors are compounds that reduce S6K activity, e.g., S6K1 or S6K2 activity. For example, compounds that inhibit S6K enzymatic activity typically bind to an ATP binding site in S6K or bind to a catalytic domain of S6K. The compound preferentially inhibits S6K1 compared to S6K2 or other S6K isoforms, given the difference in phenotypes observed between S6K1 and S6K2 knock out mice. Thus, although compounds that inhibit S6K2 or both S6K1 and S6K2 (such as rapamycin, its derivatives or other mTOR inhibitors) may be useful, the selective inhibition of S6K1 is desirable for the treatment of excessive fat accumulation, such as in obesity. Therefore, S6K1 inhibitors will typically reduce S6K1 activity by at least 10%, more preferably 20%, 50%, 100% and 200% compared to the level of reduction of S6K2 activity. Control assays may therefore be carried out, for example with immunoprecipitated S6K2 and compared to immunoprecipitated S6K1 to establish the selectivity of a modulator.

The screening methods of the invention may optionally further comprise a functional assay, comprising detecting an effect of the modulator on fat accumulation, fat metabolism or adipocyte size by said agent. Suitable methods are set out in Examples below.

The screening methods may optionally include the step of administering a potential modulator to a non-human animal having an S6 kinase gene and determining whether weight loss or gain (in particular fat accumulation) is affected relative to when the compound is absent. The non-human animals will typically be laboratory mammals such as mice or rats and various doses can be administered orally mixed with feed or by any other appropriate means, which may be chosen dependent on the properties of the compound, such as stability and targeted delivery. The S6 kinase gene may be from a different species as the laboratory mammal, for example, the use of a mouse comprising a human S6K gene, which replaces the mouse S6K gene, will be particularly useful to determine the effects of agents on human S6K without using human subjects.

The potency and efficacy of compounds for inhibition of S6K1 can also be assessed using an animal model for obesity (e.g. ob/ob mice) and compared with S6K1 knock out mice.

Kits useful for screening such compounds may also be prepared in accordance with the invention, and will comprise essentially S6K or a fragment thereof useful for screening, and instructions. Typically the S6K polypeptide will be provided together with means for detecting S6K activity and at least one compound (putative agent) or other substance described herein useful for carrying out the screening methods.

S6K for use in kits according to the invention may be provided in the form of a protein, for example in solution, suspension or lyophilised, or in the form of a nucleic acid sequence permitting the production of S6K or a fragment thereof in an expression system, optionally in situ.

Compounds (e.g., putative agents) may be inorganic or organic, for example, an antibiotic, antibody, polypeptide or peptide, and are typically isolated or purified. An "isolated" or "purified" composition is substantially free of cellular material or other contaminating proteins from the cell or tissue source from which it is derived, or substantially free from chemical precursors or other chemicals when chemically synthesized. A polypeptide that is substantially free of cellular material includes preparations of the polypeptide in which the polypeptide is separated from cellular components of the cells from which it is isolated, e.g., the polypeptide is recombinantly produced. Preferably, a preparation of a therapeutic compound, e. g., an S6K inhibitor, is at least 75%, more preferably 80%, more preferably 85%, more preferably 90%, more preferably 95%, more preferably 98%, and most preferably 99 or 100% of the dry weight of the preparation. Mixtures of compounds may also be tested during initial stages of screening.

Compounds may therefore include antibodies, preferably monoclonal antibodies, that are specific for S6K, in particular S6K1, in the sense of being able to distinguish between the polypeptide it is able to bind and other polypeptides of the same species for which it has no or substantially no binding affinity (e.g. a binding affinity of at least about 1000x worse). Specific antibodies bind an epitope on the molecule that is either not present or is not accessible on other molecules. For example, for isoform-specific inhibition (selective inhibition of S6K1 activity over S6K2 activity), the antibody may interfere with the C-terminal domain of S6K, which is not highly conserved between S6K1 and S6K2. Antibodies may be obtained using techniques which are standard in the art. For example, antibodies may be obtained from immunised animals using any of a variety of techniques known in the art, and screened, preferably using binding of antibody to antigen of interest. For instance, Western blotting techniques or immunoprecipitation may be used (Armitage et al, Nature, 357:80-82, 1992).

As an alternative or supplement to immunising a mammal with a peptide, an antibody specific for a protein may be obtained from a recombinantly produced library of expressed immunoglobulin variable domains, e.g. using lambda bacteriophage or filamentous bacteriophage which display functional immunoglobulin binding domains on their surfaces; for instance see WO92/01047.

Antibodies may be modified in a number of ways and include antibody fragments, derivatives, functional equivalents and homologues of antibodies, including synthetic molecules and molecules whose shape mimics that of an antibody enabling it to bind an antigen or epitope. Example antibody fragments, capable of binding an antigen or other binding partner are the Fab fragment consisting of the VL, VH, Cl and CHl domains; the Fd fragment consisting of the VH and CHl domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

Humanized antibodies in which CDRs from a non-human source are grafted onto human framework regions, typically with the alteration of some of the framework amino acid residues, to provide antibodies which are less immunogenic than the parent non-human antibodies, are also included within the present invention

As is apparent to one of skill in the art, a monoclonal antibody may be subjected to the techniques of recombinant DNA technology to produce other antibodies or chimeric molecules that retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB-A-2188638 or EP-A-0239400. Cloning and expression of chimeric antibodies are described in EP-A-0120694 and EP-A-0125023.

Peptides can also be used as inhibitors of S6K activity, such as a synthetic peptide containing a putative autoinhibitory domain (S6 kinase 1 residues 400-432; Flowtow and Thomas, 1992) or indeed the synthetic substrates referred to above (e.g., S6 230-249, Ala 235), which can further be used to model new inhibitors.

Alternatively, S6K activity in a cell can be reduced using nucleic acids, e.g. by pre- or post-transcriptional silencing. Thus, S6K sequences (in particular sequences specific to S6K1) may be inserted into the vectors as described above in an antisense orientation in order to provide for the production of antisense RNA or ribozymes.

Nucleic acid sequences selective for S6K1 over S6K2 include those encoding amino acids 33 -77 and amino acids 454-525 (numbering refers to that of SEQ ID NO:3 in US2003/0083284, which is hereby incorporated by reference) or portions thereof, which are typically at least 15, 18 or more nucleotides in length. Sequence comparisons may be made essentially as described above using FASTA and FASTP (see Pearson & Lipman, 1988. Methods in Enzymology 183: 63-98) to establish specificity of the sequences.

An alternative to anti-sense is to use double stranded RNA (dsRNA), which has been found to be even more effective in gene silencing than antisense alone (Fire A. et al Nature, Vol 391, (1998)). dsRNA mediated silencing is gene specific and is often termed RNA interference (RNAi) (See also Fire (1999) Trends Genet. 15: 358-363, Sharp (2001) Genes Dev. 15: 485-490, Hammond et al. (2001) Nature Rev. Genes 2: 1110-1119 and Tuschl (2001) Chem. Biochem. 2: 239-245).

RNA interference is a two-step process. First, dsRNA is cleaved within the cell to yield short interfering RNAs (siRNAs) of about 21-23nt length with 5' terminal phosphate and 3' short overhangs (∼2nt). The siRNAs target the corresponding mRNA sequence specifically for destruction (Zamore P.D. Nature Structural Biology, 8, 9, 746-750, (2001). Thus in one embodiment, the inhibition is achieved using double stranded RNA comprising an S6K-encoding sequence, in particular a sequence selective for S6K1, which may for example be a double stranded RNA (which will be processed to siRNA, e.g., as described above). Cellular defense mechanisms, such as the PKR pathway will typically need to be circumvented, for example using siRNA directed against the individual components. These RNA products may be synthesised in vitro, e.g., by conventional chemical synthesis methods.

However, to avoid the PKR pathway, chemically synthesized siRNA duplexes of about 21-23 nucleotides in length with 3'-overhang ends are preferably used (Zamore PD et al Cell, 101, 25-33, (2000)). Synthetic siRNA duplexes have been shown to specifically suppress expression of endogenous and heterologeous genes in a wide range of mammalian cell lines (Elbashir SM. et al. Nature, 411, 494-498, (2001)).

Thus, siRNA duplexes containing between 20 and 25 bps, more preferably between 21 and 23 bps, of the S6K sequence, in particular sequences selective for S6K1 over S6K2, include form one aspect of the invention e.g. as produced synthetically, optionally in protected form to prevent degradation.

Alternatively siRNA may be produced from a vector, in vitro (for recovery and use) or in vivo. Accordingly, the vector may comprise a nucleic acid sequence encoding S6K (including a nucleic acid sequence encoding a variant or fragment thereof), suitable for introducing an siRNA into the cell in any of the ways known in the art, for example, as described in any of references cited herein, which references are specifically incorporated herein by reference.

In one embodiment, the vector may comprise a nucleic acid sequence according to the invention in both the sense and antisense orientation, such that when expressed as RNA the sense and antisense sections will associate to form a double stranded RNA. This may for example be a long double stranded RNA (e.g., more than 23nts), which may be processed in vitro with Dicer to produce siRNAs (see for example Myers (2003) Nature Biotechnology 21:324-328) or siRNA, hairpin structures. Alternatively, the sense and antisense sequences are provided on different vectors. These vectors and RNA products are useful, for example, to inhibit de novo production of the S6K polypeptide in a cell. Such nucleic acids and vectors can be introduced into a host cell or administered to a mammal in a suitable form.

Thus, nucleic acids, such as siRNA can be administered to inhibit S6K1 activity. SiRNA technology can be routinely applied based on sequences specific for S6K1, such as AGTGTTTGACATAGACCTG (SEQ ID NO:1) or preferably AAGGGGGCTATGGAAAGGTTT (SEQ ID NO:2). Targeted expression of siRNAs can be achieved using tissue-specific promoters, such as promoters specific for adipose tissue or liver.

For ease of administration, however, the compound is preferably a small molecule, which might bind to the catalytic site or ATP binding site. For isoform-specific inhibition, the compound may interfere with the C-terminal domain of S6K, which is not highly conserved between S6K1 and S6K2.

In order to potentially improve S6K modulators, isolated S6K can be used to establish secondary and tertiary structure of the whole protein or at least of the areas responsible for the enzymatic activity. Conventional methods for the identification of the 3-dimensional structure are, for example, X-ray studies or NMR studies. The data obtained with these or comparable methods may be used directly or indirectly for the identification or improvement of modulators of S6K, such as to provide selectivity between S6K1 and S6K2. A commonly used method in this respect is, for example, computer aided drug design or molecular modelling.

Compounds according to the invention may be identified by screening using the techniques described hereinbefore, and prepared by extraction from natural or genetically modified sources according to established procedures, or by synthesis, especially in the case of low molecular weight chemical compounds. Proteinaceous compounds may be prepared by expression in recombinant expression systems, for example a baculovirus system, or in a bacterial system. Proteinaceous compounds are mainly useful for research into the function of signalling pathways, although they may have a therapeutic application, such as humanized inhibitory antibodies directed against S6 kinase 1.

Low molecular weight compounds, on the other hand, are preferably produced by chemical synthesis according to established procedures. They are primarily indicated as therapeutic agents. PKC inhibitors, or derivatives or modifications thereof, may be used as potential agents effective in selectively inhibiting S6K1 and in treating obesity or other weight disorders dependent on fat accumulation. Low molecular weight compounds and organic compounds in general may be useful as agents for use in the treatment of obesity (or conditions associated with weight disorders).

The present invention also provides a method for reducing adipocyte size comprising contacting an adipocyte with an effective amount of an S6 kinase 1 inhibitor. Thus, also provided by the invention are compounds that directly modulate S6 kinase activity for use in treating weight disorders dependent on fat accumulation, such as obesity. In particular, compounds that selectively inhibit S6 kinase 1 activity over S6K2 activity for use in treating overweight conditions, such as obesity are provided, or for treating mammals such as humans at risk of becoming obese.

Women who are overweight or obese prior to menopause are thought to have a predisposition to breast cancer. Without wishing to be bound by theory, the present inventors believe the increased adipose tissue in individuals with a Higher Mass Index can provide energy to drive cancer mass growth and thus individuals with a higher Body Mass Index are at risk of developing cancer in general. Thus, compounds that selectively inhibit S6K1 activity over S6K2 activity may be useful in treating a predisposition to cancer or a cancerous condition in obese individuals in need of such treatment. Thus the description herein referring to the treatment of obesity also applies to conditions associated with the weight disorders, such as cancerous conditions (e.g., breast cancer) in overweight or obese individuals.

Obesity is normally defined through the determination of the Body Mass Index (BMI), which is calculated using a simple formula (weight in kg divided by the square of height in meters). A calculated BMI of 18.5 to 24.9 is considered to be normal weight; a BMI of 25-29.9 kg/m2 indicates overweight; a BMI of 30-39.9 indicates obesity; and a BMI of 40 and upwards indicates morbid obesity. Similarly, a calculated BMI of less than 18.5 may be considered an underweight condition. Alternatively, waist circumference (estimates fat distribution), waist-to-hip ratio (estimates fat distribution), skinfold thickness (if measured at several sites, estimates fat distribution), or bioimpedance (based on the principle that lean mass conducts current better than fat mass, i. e., fat mass impedes current, estimates % fat) can be measured. Overweight individuals are characterized as having a waist circumference of > 94 cm for men or > 80 cm for women and waist to hip ratios of > 0.95 in men and > 0.80 in women. Obese individuals are characterized as having a waist circumference > 102 cm (40 inches) for men or 88 cm (35 inches) for women.

S6K modulators (e.g., inhibitors) for use in treating weight disorders dependent on fat accumulation (e.g. overweight conditions, such as obesity) may thus be formulated as medicaments according to conventional methodology, depending on the exact nature of the modulator, and will typically comprise the modulator or a precursor thereof in association with a biologically acceptable carrier. In considering various therapies, it is understood that such therapies may be targeted to tissues demonstrated to express S6K1, in particular to adipocytes.

Compounds are administered at a dose that is therapeutically effective. The term "therapeutically effective amount" as used herein means that the amount of a compound (s) or pharmaceutical composition elicits a beneficial biological or medicinal response in a tissue, system, animal or human. For example, a therapeutically effective amount of an S6K1 inhibitory compound is a dose which leads to a clinically detectable improvement in weight loss resulting from loss of fat tissue.

Treatment includes the management and care of an individual for the purpose of alleviating a symptom of a weight disorder dependent on fat accumulation. Treatment includes the administration of a compound to prevent the onset of symptoms or complications of the disorder, alleviating the symptoms or complications, or eliminating the condition or disorder.

Delivery of the modulator to the affected cells and tissues can be accomplished using appropriate packaging or administration systems. For example, the modulator may be formulated for therapeutic use with agents acceptable for pharmaceutical administration and delivered to the subject by acceptable routes to produce a desired physiological effect. An effective amount is that amount that produces the desired physiological effect, such as, weight loss.

In a further aspect of the invention, the invention also provides a modulator (e.g., selective inhibitor of S6 kinase 1) for the manufacture of a medicament for the treatment or prophylactic treatment of a weight disorder dependent on fat accumulation (e.g., obesity). Suitable modulators, in particular inhibitors, such as those identified in the functional or other assays discussed above, may be incorporated into medicaments e.g. after further testing for toxicity. Thus the relevant methods may include the further step of formulating a selected modulator as a medicament for a disease e.g. in which it is desired to control weight disorders dependent on fat accumulation. Such inhibitors and medicaments for use in the treatment of these diseases, and methods of treatment comprising their use form further aspects of the invention.

The compositions may include, in addition to the above constituents, pharmaceutically-acceptable excipients, preserving agents, solubilizers, viscosity-increasing substances, stabilising agents, wetting agents, emulsifying agents, sweetening agents, colouring agents, flavouring agents, salts for varying the osmotic pressure, buffers, or coating agents. Such materials should be nontoxic and should not interfere with the efficacy of the active ingredient. The precise nature of the carrier or other material may depend on the route of administration. Examples of techniques and protocols can be found in "Remington's Pharmaceutical Sciences", 16th edition, Osol, A. (ed.), 1980.

Where the composition is formulated into a pharmaceutical composition, the administration thereof can be effected parentally such as orally, nasally (e.g. in the form of nasal sprays) or rectally (e.g. in the form of suppositories). However, the administration can also be effected parentally such as intramuscularly, intravenously, cutaneously, subcutaneously, or intraperitoneally (e.g. in the form of injection solutions).

Thus, for example, where the pharmaceutical composition is in the form of a tablet, it may include a solid carrier such as gelatine or an adjuvant. For the manufacture of tablets, coated tablets, dragees and hard gelatine capsules, the active compounds and their pharmaceutically-acceptable acid addition salts can be processed with pharmaceutically inert, inorganic or organic excipients. Lactose, maize, starch or derivatives thereof, talc, stearic acid or its salts etc. can be used, for example, as such excipients for tablets, dragees and hard gelatine capsules. Suitable excipients for soft gelatine capsules are, for example, vegetable oils, waxes, fats, semi-solid and liquid polyols etc. Where the composition is in the form of a liquid pharmaceutical formulation, it will generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may also be included. Other suitable excipients for the manufacture of solutions and syrups are, for example, water, polyols, saccharose, invert sugar, glucose, trihalose, etc. Suitable excipients for injection solutions are, for example, water, alcohols, polyols, glycerol, vegetable oils, etc. For intravenous, cutaneous or subcutaneous injection, or intracatheter infusion into the brain, the active ingredient will be in the form of a parenterally-acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers and/or other additives may be included, as required.

Also provided are methods of evaluating treatments for weight disorders dependent on fat accumulation, the method comprising administering a therapeutic agent (for example, identified by the screening methods provided herein) to a non-human animal comprising an S6 kinase gene, in particular S6K1, and determining the effect of the agent on weight gain or loss. Alternatively, a sample, such as adipose tissue or peripheral blood can be withdrawn and tested for S6K levels or activity levels to establish a modulatory effect on S6K.

In addition, methods of evaluating treatments for weight disorders are provided, comprising administering a therapeutic agent to a non-human animal deficient in S6 kinase (such as a knock-out animal), in particular deficient in S6K1, and determining the effect of the agent. Such methods can be used to establish whether any unwanted side effects of the therapeutic agent are present.

The present inventors have shown that wild type mice fed a high fat diet have strikingly elevated S6K1 activity, as do ob/ob mice, a genetic mouse model of obesity (see Example 9). The invention therefore also provides a method of diagnosing a predisposition to a weight disorder, comprising: obtaining a sample from an individual, detecting the level of S6 kinase, preferably S6 kinase 1, in the sample and correlating a change in the amount of S6 kinase in the sample when compared to a normal control value or range of values with a predisposition to a weight disorder. The presence of S6 kinase can be easily determined using antibodies or using activity assays as described above. S6K expression can also be detected at the transcript level, e.g., using PCR techniques. When protein levels are detected, antibodies specific for S6K2 can be used as a control when S6K1 specific measurements are desired. In general, an increase in S6 kinase 1 activity of at least 10%, preferably at least 20%, 30%, 40% or 50% when compared to a normal control value or range of values is indicative of a predisposition to obesity. Most preferably, the increase in activity is at least 2-fold than that of a control value. The sample may be any tissue sample or body fluid, but is preferably adipose tissue.

To determine the kinase activity of S6K 1 (independent of other S6K isoforms such as S6K2), a sample is obtained from a test subject. Cells present in the sample can be lysed and proteins extracted. Optionally, further purification steps can be carried out. The sample can then be subjected to immunoprecipitation using an S6K1 specific antibody, which are known in the art. Following immunoprecipitation of S6K1, a standard kinase assay can be performed as described above.

The invention is further described, for the purposes of illustration only, in the following examples.

### EXAMPLES

Methods of molecular genetics, protein and peptide biochemistry and immunology referred to but not explicitly described in this disclosure and examples are reported in the scientific literature and are well known to those skilled in the art. For example, standard methods in genetic engineering are carried out essentially as described in Sambrook et al., Molecular Cloning: A laboratory manual, 2nd Ed., Cold Spring Harbor Laboratory Press, Cold Spring Harbor NY, 1989.

### Example 1:S6K1 deficient mice are smaller than wild type

S6K1 deficient mice were previously shown to have a reduction in body size during embryogenesis but the effect was thought to be mostly overcome by adulthood, diminishing from 20 to 15% by 11 weeks of age. This Example demonstrates that as they age, S6K1 deficient mice maintain a lower body weight relative to wild type mice. Male mice on a normal chow diet (NCD, 4% of total calories derived from fat, 3035 kcal kg-1, KLIBA-NAFAG, Switzerland) were followed over a period of seventeen weeks from ten weeks of age.

S6K1 deficient mice were generated as described by Shima et al. (1998, EMBO J., 17, 6649-6659). Mice were kept on a hybrid background derived from the C57BI/6 and 129Oia mouse strains, housed in groups of 12 (in cages of 3) and maintained on a 12 hour light/12 hour dark cycle (lights on at 06:00 GMT). Body weight was recorded weekly in wild type (wt) and S6K1 deficient mice fed regular chow diet. Unexpectedly, the results showed that the rate at which S6K1-/- mice gained weight was much slower than that of wild mice, such that the difference in weight at twenty-seven weeks, as compared to ten weeks, had increased to 25%. It should also be noted S6K1-/- mice displayed little variation in weight as compared to wild type (wt) mice.

The data are provided in Tables I and II below and show that S6K1 deficient mice are smaller and have lower body weight compared to wt.

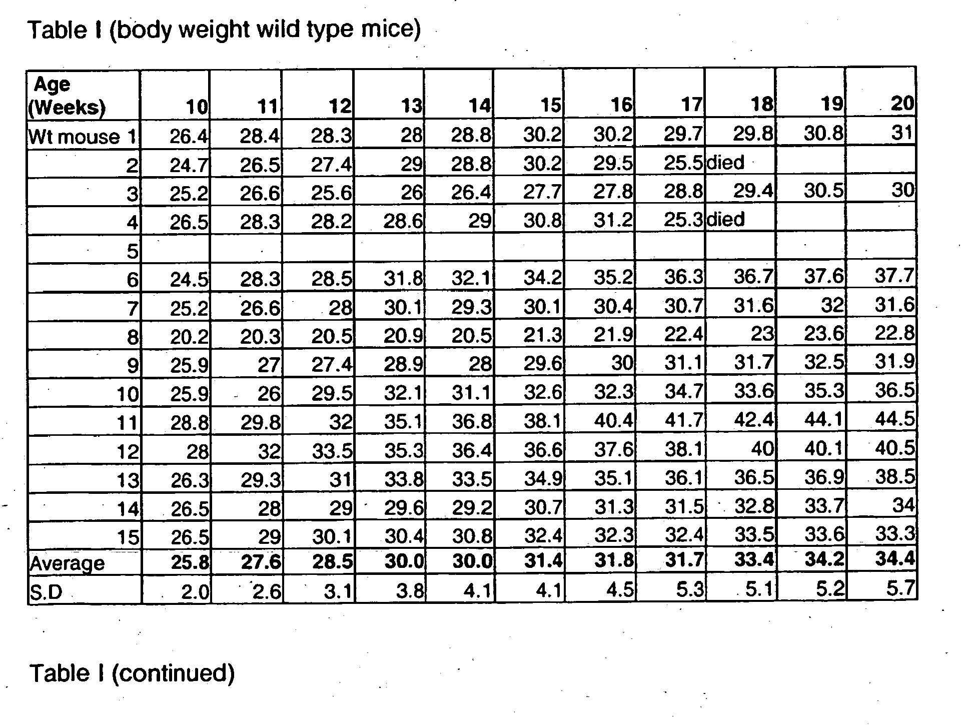

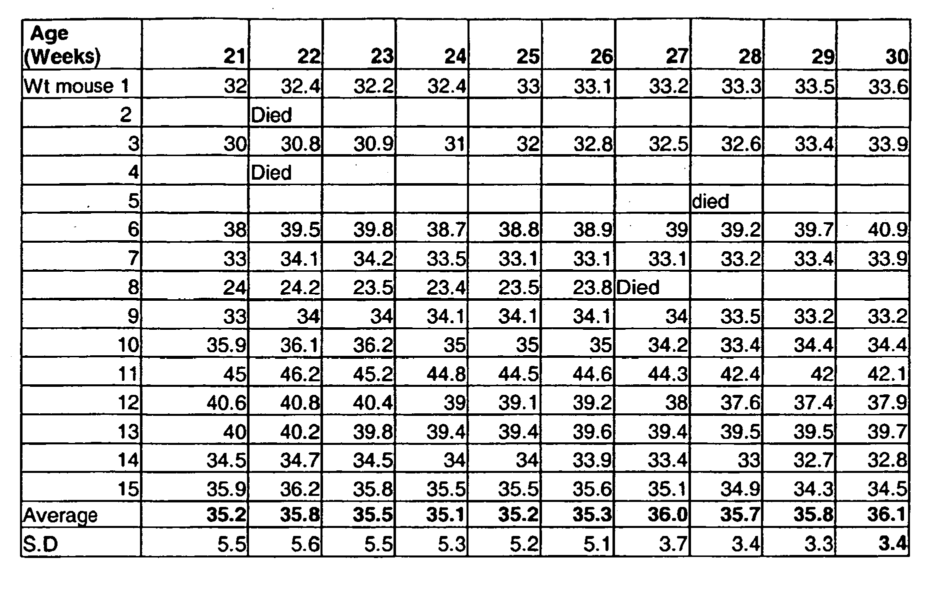

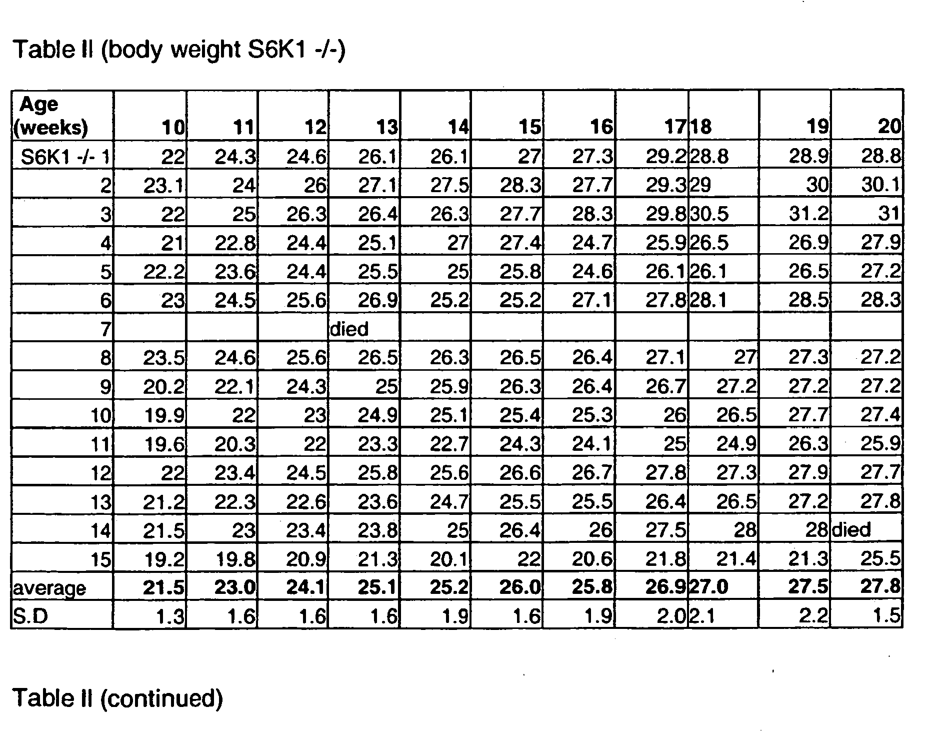

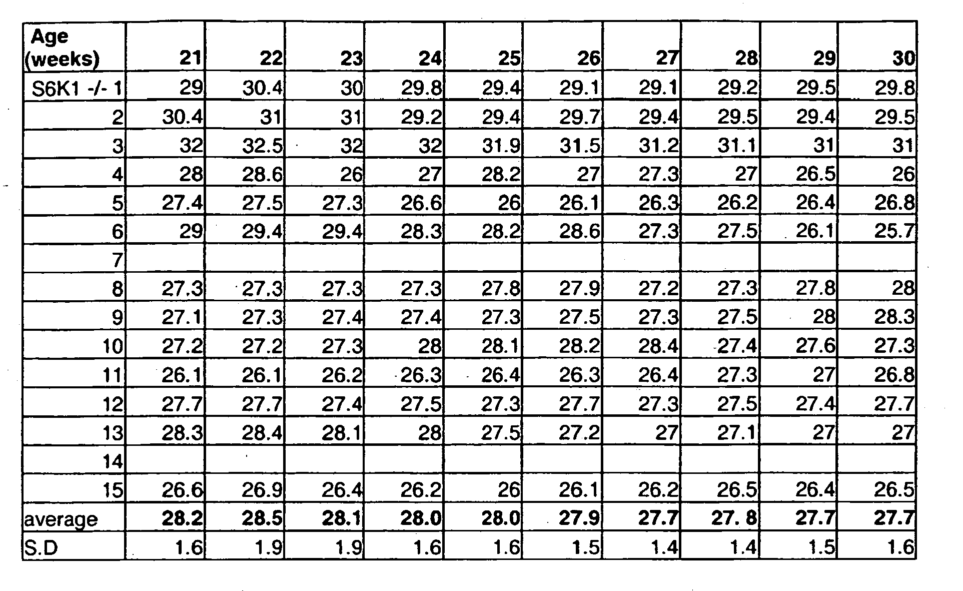

### Example 2:S6K1 deficient mice have reduced body fat

Mice were dissected to determine the cause of the lower body weight exhibited by S6K1 deficient mice described in Example 1. S6K1 deficient mice were shown to have less intra-abdominal fat pads. Each fat mass and organ mass was weighed from tissue removed from 6 -month old male mice. Dissection of S6K1 mice revealed a severe reduction of epididymal white adipose tissue fat relative to wild type mice (0.8% +/- 0.1 % compared with 3.4% +/- 0.1 %; the values are averaged values +/- standard error mean; S.E.M). Percentage brown adipose tissue/body weight was also reduced in S6K1 deficient mice (0.5% +/- 0.05 % compared with 1:0 +/- 0.1 %), whereas organ size was essentially unaffected (see Table III below). Similar results were found also in female mice. The decrease in fat was not associated with a selective decrease in the deposition of fat in liver or muscles.

**Table III**

| **Tissue** | **Wild-type** | **S6K1 -/-** |
|---|---|---|
| | (tissue weight/body( weight) | (tissue weight/body weight) |
| Liver | 4.2+/-0.3% | 4.6+/- 0.2% |
| Thymus | 0.14+/-0.014% | 0.18+/-0.01 % |
| Muscle | 0.5+/-0.02% | 0.5+/-0.01 % |
| Kidney | 1.5+/-0.05% | 1.9+/-0.07% |
| Lung | 0.8+/-0.1 % | 0.8+/-0.1 % |
| Heart | 0.8+/-0.1 % | 0.7+/-0.05% |
| Testis | 0.6+/- 0.02% | 0.75+/- 0.04% |
| Spleen | 0.33+/-0.05% | 0.28+/-0.02% |
| Brain | 1.25+/-0.03% | 1.63+/-0.06% |

The data establish that S6K1 -/- mice have reduced body white fat and brown fat relative to wild type mice.

### Example 3: S6K1 deficient adipocytes are smaller

To establish why S6K1 deficient mice exhibit less fat, adipose tissue sections were stained with hematoxylin and eosin and visualized by 20-fold magnification using a histology microscope. Both epididymal white adipose tissue (WAT) and brown adipose tissue from S6K1 deficient mice exhibited smaller cell size when compared to wild type.

Cell density and size was quantified in the adipose tissue sections of WAT. Cell numbers were counted within an area of 120 x 120mm on six sections from three individual animals for each genotype using ImageProPlus software (Media Cybernetics). The data is presented below in Table IV:

**Table IVa (wild type cell size distribution)**

| **Cell size** µ**m2** | **Cell number** |
|---|---|
| 0-99 | |
| 100-199 | 1 |
| 200-299 | 0 |
| 300-399 | 0 |
| 400-499 | 3 |
| 500-599 | 3 |
| 600-699 | 9 |
| 700-799 | 6 |
| 800-899 | 7 |
| 900-999 | 12 |
| 1000-1999 | 157 |
| 2000-2999 | 136 |
| 3000-3999 | 122 |
| 4000-4999 | 87 |
| 5000-5999 | 63 |
| 6000-6999 | 41 |
| 7000-7999 | 25 |
| 8000-8999 | 21 |
| 9000-9999 | 4 |
| >10000 | 6 |

**Table IVb (wild type cell size distribution)**

| **cell size** µ**m2** | **cell number** |
|---|---|
| 0-99 | |
| 100-199 | 1 |
| 200-299 | 0 |
| 300-399 | 0 |
| 400-499 | 3 |
| 500-599 | 3 |
| 600-699 | 9 |
| 700-799 | 6 |
| 800-899 | 7 |
| 900-999 | 12 |
| 1000-1199 | 30 |
| 1200-1399 | 30 |
| 1400-1599 | 42 |
| 1600-1799 | 28 |
| 1800-1999 | 27 |
| 2000-2999 | 136 |
| 3000-3999 | 122 |
| 4000-4999 | 87 |
| 5000-5999 | 63 |
| 6000-6999 | 41 |
| 7000-7999 | 25 |
| 8000-8999 | 21 |
| 9000-9999 | 4 |
| >10000 | 6 |

**Table IVc (S6K1 -/- cell size distribution)**

| **cell size µm2** | **cell number S6K 1 ko** |
|---|---|
| 0-99 | 9 |
| 100-199 | 14 |
| 200-299 | 15 |
| 300-399 | 20 |
| 400-499 | 30 |
| 500-599 | 24 |
| 600-699 | 41 |
| 700-799 | 43 |
| 800-899 | 42 |
| 900-999 | 48 |
| 1000-1999 | 181 |
| 2000-2999 | 33 |
| 3000-3999 | 1 |
| 4000-4999 | |
| 5000-5999 | |
| 6000-6999 | |
| 7000-7999 | |
| 8000-8999 | |
| 9000-9999 | |
| >10000 | |

**Table IVd**

| | **wt** | **S6K1ko** |
|---|---|---|
| Average | 3556 | 1001 |
| % of weight | 1 | 0.28 |

Analysis of the size of fat cells in epididymal fat pads by either scanning electron microscopy or by hematoxyline-eosine staining showed a striking reduction in cell size, with quantitation revealing that adipocytes from S6K1-/- mice were 71% smaller than those of wild type mice (wt: 3129 ± 904 , n=3; S6K1-/-: 918 ± 189 mm2 , n=3, P< 0.05), with many adipocytes exhibiting a multilocular phenotype. These studies also revealed that the distribution of cell size, like body weight, was much more homogeneous in S6K1-/- mice as compared to wild type mice and a rough calculation of cell surface areas versus total mass suggested that there was little effect on cell number. In summary, results from electron microscopy, histology and cell density/size analysis establishes that the reduction in fat in S6K1 deficient animals is due to a reduction in fat cell size.

### Example 4: Diet is not the cause of less fat in S6K1 deficient mice

To establish whether S6K1 mice exhibited dietary differences over wild type mice, which would explain the reduction in fat in S6K1 mice, food intake per mouse was measured every other day for 15 days using normal or high fat chow. Irrespective of whether placed on normal or high fat diet, S6K1 deficient mice eat the same total amount of food as wild type (about 4.6 +/- 0.1 g food/mouse/day), but compared to body weight, they eat much more (about 17% or more, or 0.18 compared to 0.15 g food/g body weight/day).

### Example 5: S6K1 deficient mice exhibit an enhanced metabolic rate

The increased food uptake, combined with reduced WAT, raised the possibility of enhanced metabolic activity. The metabolic rate of S6K1 deficient and wild type mice were examined by indirect calorimetry to monitor oxygen consumption and carbon dioxide production every 15 min over an eight-hour fasting period employing an Oxymax (Columbus Instruments, Columbus, OH).

The results show a striking 27% increase in the rate of oxygen consumption in S6K1-/- mice versus wild type mice through out the experiment. Calculation of the respiratory exchange ratio (RER = ratio of CO2 produced to 02 consumed) gave values of 0.713 ± 0.004 for wild type mice and 0.709 ± 0.003 for S6K1-/- mice; P< 0.01), arguing that both animals were largely utilizing fatty acids as an energy source.

Metabolite assays were carried out as follows. Blood was collected from retroorbital sinus after overnight fast or 1 hr after beginning of the meal followed by overnight fast. Nonesterified fatty acids, and triglycerides were determined by enzymatic assays (Boehringer-Mannheim, Germany). Plasma leptin was measured using Rat leptin RIA kit (Linco Research, St Louis, MO.). The results are shown in Table V. Plasma leptin levels were significantly reduced in S6K1-/- mice (Table V) in keeping with the mice's increased consumption of food relative to body weight.

**Table V one-hour postprandial triglycerides, free fatty acids and leptin levels after overnight fasting of 6 month old male wild type, and S6K1 deficient mice fed a normal, or high fat diet.**

| Diet | WT | | S6K1 -/- | |
|---|---|---|---|---|
| | Normal | High Fat | Normal | High Fat |
| | | | | |
| Triglycerides(mmol/l) | 0.61 ± 0.09 | 1.08 ± 0.15 | 0.55 ± 0.06 | 0.95 ± 0.14 |
| Free fatty acids (mmol/l) | 0.26 ± 0.05 | 0.27 ± 0.02 | 0.19 ± 0.04 | 0.56 ± 0.10* |
| leptin (ng/ml) | 5.50 ± 0.82 | 12.6 ± 0.00 | 3.42 ± 0.75 | 6.34 ± 2.35* |

| | | | | |
|---|---|---|---|---|
| Data represent means ± s.e.m. *, P< 0.05 compared with wild type (n=6 to 18). P values were calculated by two-tailed, unpaired Student's *t*-test. | | | | |

Although not wishing to be bound by theory, given the reduced adipose tissue mass, increased metabolic rate, and the fact that when corrected for body weight, plasma triglycerides and free fatty acids were similar between genotypes (Table V), it was reasoned that in the absence of S6K1, either triglycerides in adipose tissue were being rapidly utilized or that the free fatty acids never reached adipose tissue for storage, but were immediately taken up by muscle and oxidized.

Although WAT is not normally an energy consuming tissue, that there was no difference in the basal levels of circulating fatty acids in S6K1-/- mice, as compared to wild type mice (Table V), suggested that free fatty acids may be directly oxidized in WAT. Consistent with this hypothesis, electron micrographs revealed the presence of many multilocular adipocytes, which displayed a dramatic increase in the size and number of mitochondria, phenotypes completely absent in adipocytes from wild type mice. Others have shown that overexpression of UCP1 in WAT induces a similar phenotype, and UCP1 levels, measured by quantitative real time-PCR, were dramatically upregulated in WAT from S6K1-/- mice as compared to WAT from wild type mice.

### Example 6: S6K1 deficient mice exhibit increased basal lipolysis

The breakdown of triglycerides in adipose tissue (lipolysis) was measured by monitoring the release of either fatty acids or glycerol from mature adipocytes. Primary adipocytes were prepared from epididymal fat pads by collagenase digestion as described previously (Marette et al., 1991). Cells were incubated for 30 min at 37°C with or without norepinephrine (Sigma-Aldrich SARL, St-Quentin Fallavier, France) at different concentrations (10⁻⁸ to 10⁻⁵M). Norepinepherine is a beta-adrenergic agonist and stimulates the breakdown of adipocyte triglyceride to glycerol and free fatty acids (lipolysis) and increases the basal metabolic rate (thermogenesis). Despite the reduction in cell size of epididymal adipocytes (see Example 3), the results showed that the basal rate of fatty acid release was approximately 5-fold higher in adipocytes from S6K1-/- mice as compared to wild type mice. The rate of fatty acid release increased in both genotypes in a dose dependent manner upon the addition of norepinephrine, reaching similar maximum values, with the increase much steeper in wild type mice. Similar results were obtained for the release of glycerol. Therefore, S6K1-/- mice are protected against fat accumulation partly due to a sharp increase in basal lipolysis.

### Example 7: S6K1 deficient mice exhibit impaired adipogenesis

During the isolation of mature adipocytes for the lipolysis studies, it became evident that there were few preadipocytes present in epididymal WAT of S6K1-/- mice. This, along with the inability of adipocytes to store triglycerides, prompted experiments to compare the ability of mouse embryonic fibroblasts (MEFs) from S6K1-/- and wild type embryos to differentiate into adipocytes using an adipocyte differentiation mix.

Briefly, adipocyte differentiation was induced essentially as previously described (Hansen et al., 1999, J. Biol. Chem., 274, 2386-2393) using wild type or S6K1 deficient mouse embryonic fibroblasts (MEFs). The passage number of MEFs was within one passage. For differentiation, 2-day post confluent cells (day 0) were treated with growth medium containing 1 µM dexamethasone (Sigma), 0.5 mM methylisobutylxanthine (Aldrich), 5 µg/ml insulin (Boehringer Mannheim), and Ciglitazone (thiazolinedione, PPAR agonist: BIOMOL, GR-205, 0.5 µM) for 2 days. From day 2, the medium contained 5 µg/ml insulin and Ciglitazone and renewed every other day. Oil red O staining: Oil red O staining solution (0.5% Oil red O in isopropyl alcohol solution-distilled water (60:40) was filtered. Cells were washed with PBS and stained for 30 min and then washed with distilled water two times. Cells deficient in S6K1 showed much less staining. Therefore, MEFs lacking S6K1 had a reduced adipogenic potential, as evaluated by the reduced Oil Red O lipid staining, consistent with lower levels of aP2 mRNA. Taken together the results suggest that S6K1-/- mice have reduced WAT because of impaired adipogenesis and an inability to store fat.

### Example 8: S6K1 deficient mice are protected against diet-induced obesity

The failure of S6K1-/- mice to accumulate fat with age combined with their overall increase in metabolic rate suggested that they may be protected against diet-induced obesity. In this example, body weight was recorded weekly in wild type and S6K1 deficient mice fed high fat diets (HFD, 60% of total calories derived from fat, 4057 kcal kg-1, Research diets, USA). When S6K1 mice are placed on a high fat diet, absolute body weight gain of S6K1 deficient mice was about 10.5 g over the period of high fat diet feeding (from week 7 to 27 of age) compared to about 14.4 g in wild type mice. Similar to the situation on the NCD (Example 1), S6K1-/- mice displayed little variation in weight on a HFD as compared to wild type mice. Given the smaller body size of the knockout, relative weight gain was similar between genotypes (58.9% increase of body weight in S6K1 deficient mice compared to 58.0% increase of body weight in wild type after high fat diet feeding for 5 months. Even though the relative percentage of body weight gain in S6K1 deficient mice was similar to wild type, they fail to put on fat to the same extent as wild type mice. Over a three-month period, wild type mice gained 0.1 g/g compared with 0.02 g/g fat/body weight by S6K1 deficient mice.

Mice of both genotypes consumed less food on a HFD than on a NCD, probably due to the higher caloric density of the HFD as compared to the NCD. Although in absolute terms, S6K1-/- mice consume the same amount of food as wild type mice, when normalized to body weight they consume 44% more food. Thus, even though S6K1 mice eat more, they do not put on fat to the same degree as wild type mice.

Indirect calorimetry measurements were conducted over an eight-hour fasting period on HFD and NCD mice. In both genotypes oxygen consumption increased on the HFD, as compared to the NCD, but the effect was more pronounced for S6K1-/- mice, such that the difference between S6K1-/- mice and wild type mice increased from 25% to 30%. The data further showed that the RER remained unchanged in S6K1-/- mice on a HFD versus a NCD, 0.708 ± 0.002 vs 0.709 ± 0.004, respectively, whereas in wild type mice the RER increased from 0.713 ± 0.004 on NCD to 0.729 ± 0.002 (n=6, P< 0.01) on a HFD diet, indicative of an increase in carbohydrate relative to fatty acid oxidation. Despite the fact that S6K1-/- mice on either diet displayed a high metabolic rate, on a HFD they exhibited a threefold increase in circulating free fatty levels, whereas in wild type mice there was no significant change in free fatty acids levels (Table V). Hence, S6K1-/- mice fail to accumulate fat at an appreciable rate when challenged with a HFD.

### Example 9: Obese animals and wild type animals on high fat diet exhibit elevated S6K1 phosphorylation in adipose tissue

To examine whether S6K1 is affected in adipose tissue from normal and obese genetic models, the phosphorylation of S6K1 was detected. This can easily be carried out using phospho-specific antibodies. The level of S6K1 T389 and S6 S240/S244 phosphorylation in adipose tissue of wild type mice fasted for a short period was determined. Upon growth factor stimulation, S6 is multiply phosphorylated at the carboxy terminus on five serine residues in an ordered fashion beginning with Ser236, followed sequentially by > Ser235 > Ser240 > Ser244 and Ser247. Basal values of S6K1 T389 and S6 S240/S244 phosphorylation are low in mice fasted for a short period after being maintained on a NCD. In sharp contrast, the same mice maintained on a HFD and treated under the identical conditions, maintained high-elevated levels of S6K1 T389 and S6 S240/S244 phosphorylation.

The present inventors also examined S6K1 activity in ob/ob mice, a genetic model for obesity. The results show that the ob/ob mice maintained on NCD have elevated S6K1 T389 and S6 S240/S244 phosphorylation as compared to wild type mice on a NCD. Preliminary human data are consistent with these data and provide further support for S6K1 as a promising drug target in the treatment of patients suffering from obesity and as a potential diagnostic marker.

## Claims

1. A method of identifying an agent effective in treating weight disorders dependent on fat accumulation, said method comprising the steps of:
i) incubating S6 kinase 1 with a compound;
ii) detecting 36 kinase 1 activity; and
iii) determining a compound-induced modulation in the S6 kinase 1 activity relative to when said compound is absent, wherein an alteration of the S6 kinase 1 activity in the presence of the compound is indicative of an agent effective in treating weight disorders dependent on fat accumulation.

2. The method according to claim 1, wherein said modulation is inhibition of S6 kinase 1 activity and said weight disorder is obesity or an overweight condition.

3. The method according to claim 1, wherein said modulation is activation of S6 kinase 1 activity and said weight disorder is an underweight condition resulting from insufficient fat accumulation.

4. The method of any one of the preceding claims, comprising determining S6 kinase 1 activity using S6 as a substrate.

5. The method of claims 1 to 3, comprising determining S6 kinase 1 activity using a peptide substrate.

6. A method of screening for an agent effective in treating weight disorders, the method comprising (a) contacting ex vivo transcriptionally active cellular components with a nucleic acid encoding an S6 kinase 1 gene operably linked to a promoter sequence or an S6 kinase 1 promoter sequence operably linked to a reporter gene in the presence of at least one compound; and (b) detecting an effect of said compound on S6 kinase 1 expression or S6 kinase 1 promoter activity, wherein detection of a,decrease or an increase in S6 kinase 1 expression or promoter activity is indicative of an agent effective in treating weight disorders dependent on fat accumulation.

7. The method of claim 6, wherein said transcriptionally active cellular components and said nucleic acid is present in a cell.

8. The method of any one of the preceding claims, further comprising detecting an effect on fat accumulation, fat metabolism or adipocyte size by said agent.

9. A method of diagnosing a predisposition to a weight disorder dependent on fat accumulation, comprising:
providing a sample obtained from an individual,
detecting the level of S6 kinase 1 activity in said sample, and
correlating a change in S6 kinase 1 activity when compared to a normal control value or range of values with a predisposition to a weight disorder dependent on fat accumulation.

10. The method of claim 9, wherein said change is an increase in S6 kinase 1 activity when compared to a normal control value or range of values and said weight disorder is an overweight condition or obesity.

## Patentansprüche

1. Verfahren zum Identifizieren eines Agens mit Wirksamkeit bei der Behandlung von Gewichtsproblemen, die auf Fettakkumulation beruhen, wobei das Verfahren die folgenden Schritte umfaßt:
i) Inkubieren der S6-Kinase 1 mit einer Verbindung;
ii) Nachweisen der S6-Kinase-1-Aktivität; und
iii) Bestimmen einer durch die Verbindung induzierten Modulation der S6-Kinase-1-Aktivität im Vergleich zu Abwesenheit der Verbindung, wobei eine Veränderung der S6-Kinase-1-Aktivität in Gegenwart der Verbindung ein Agents, das sich für die Behandlung von Gewichtsproblemen, die auf Fettakkumulation beruhen, eignet, anzeigt.

2. Verfahren nach Anspruch 1, wobei es sich bei der Modulation um die Hemmung der S6-Kinase-1-Aktivität und bei dem Gewichtsproblem um Adipositas oder Übergewichtigkeit handelt.

3. Verfahren nach Anspruch 1, wobei es sich bei der Modulation um die Aktivierung der S6-Kinase-1-Aktivität und bei dem Gewichtsproblem um Untergewichtigkeit, die auf unzureichender Fettakkumulation beruht, handelt.

4. Verfahren nach einem der vorhergehenden Ansprüche, bei dem man die S6-Kinase-1-Aktivität unter Verwendung von S6 als Substrat bestimmt.

5. Verfahren nach den Ansprüchen 1 bis 3, bei dem man die S6-Kinase-1-Aktivität unter Verwendung eines Peptidsubstrats bestimmt.

6. Verfahren zum Screenen auf ein Agens mit Wirksamkeit bei der Behandlung von Gewichtsproblemen, wobei das Verfahren folgendes umfaßt: (a) ex-vivo-Inkontaktbringen von transkriptionsmäßig aktiven Zellkomponenten mit einer Nukleinsäure, die für ein S6-Kinase-1-Gen in operativer Verknüpfung mit einer Promotersequenz oder eine S6-Kinase-1-Promotersequenz in operativer Verknüpfung mit einem Reportergen in Kontakt bringt, und zwar in Gegenwart von mindestens einer Verbindung; sowie (b) das Nachweisen einer Wirkung der Verbindung auf die S6-Kinase-1-Expression oder S6-Kinase-1-Promoteraktivität, wobei der Nachweis einer erniedrigten oder erhöhten S6-Kinase-1-Expression oder -Promoteraktivität ein Agens mit Wirksamkeit bei der Behandlung von Gewichtsproblemen, die auf Fettakkumulation beruhen, anzeigt.

7. Verfahren nach Anspruch 6, wobei die transkriptionsmäßig aktiven Zellkomponenten und die Nukleinsäure in einer Zelle vorliegen.

8. Verfahren nach einem der vorhergehenden Ansprüche, das weiterhin umfaßt, daß man eine Wirkung des Agens auf die Fettakkumulation, den Fettmetabolismus oder die FettZellgröße nachweist.

9. Verfahren zum Diagnostizieren einer Anfälligkeit gegenüber einem Gewichtsproblem, das auf Fettakkumulation beruht, umfassend die folgenden Schritte:
Bereitstellen einer von einem Patienten entnommenen Probe,
Nachweisen des Ausmaßes der S6-Kinase-1-Aktivität in der Probe, und
Korrelieren einer Veränderung bei der S6-Kinase-1-Aktivität im Vergleich zu einem normalen Kontrollwert oder Bereich von Werten mit einer Anfälligkeit gegenüber einem Gewichtsproblem, das auf Fettakkumulation beruht.

10. Verfahren nach Anspruch 9, wobei es sich bei der Veränderung um eine im Vergleich zu einem normalen Kontrollwert oder Bereich von Werten erhöhte S6-Kinase-1-Aktivität und bei dem Gewichtsproblem um Übergewichtigkeit oder Adipositas handelt.

## Revendications

1. Méthode d'identification d'un agent efficace dans le traitement des troubles du poids qui dépendent de l'accumulation de graisses, ladite méthode comprenant les étapes consistant à :
i) incuber la S6 kinase 1 avec un composé ;
ii) détecter l'activité de la S6 kinase 1 ; et
iii) déterminer une modulation induite par un composé dans l'activité de la S6 kinase 1 par rapport à lorsque ledit composé est absent, une modification de l'activité de la S6 kinase 1 en présence du composé étant indicatrice d'un agent efficace dans le traitement des troubles du poids dépendant de l'accumulation de graisses.

2. Méthode selon la revendication 1, **caractérisée en ce que** ladite modulation est l'inhibition de l'activité de la S6 kinase 1 et ledit trouble du poids est l'obésité ou une surcharge pondérale.

3. Méthode selon la revendication 1, **caractérisée en ce que** ladite modulation est l'activation de l'activité de la S6 kinase 1 et ledit trouble du poids est une insuffisance pondérale provenant de l'accumulation insuffisante de graisses.

4. Méthode selon l'une quelconque des revendications précédentes, comprenant la détermination de l'activité de la S6 kinase 1 en utilisant S6 comme substrat.

5. Méthode selon les revendications 1 à 3, comprenant la détermination de l'activité de la S6 kinase 1 en utilisant un substrat peptidique.

6. Méthode de criblage d'un agent efficace dans le traitement des troubles du poids, comprenant (a) la mise en contact ex vivo de composants cellulaires transcriptionnellement actifs avec un acide nucléique codant pour un gène S6 kinase 1 lié de façon opérationnelle à une séquence promotrice ou une séquence promotrice S6 kinase 1 liée de façon opérationnelle à un gène rapporteur en présence d'au moins un composé ; et (b) la détection de tout effet dudit composé sur l'expression de la S6 kinase 1 ou l'activité du promoteur de la S6 kinase 1, la détection d'une réduction ou d'une augmentation de l'expression ou de l'activité du promoteur de la S6 kinase 1 étant indicatrice d'un agent efficace dans le traitement des troubles du poids dépendant de l'accumulation de graisses.

7. Méthode selon la revendication 6, **caractérisée en ce que** lesdits composants cellulaires transcriptionnellement actifs et ledit acide nucléique sont présents dans une cellule.

8. Méthode selon l'une quelconque des revendications précédentes, comprenant en outre la détection d'un effet sur l'accumulation de graisses, le métabolisme des graisses ou la taille des adipocytes par ledit agent.

9. Méthode de diagnostic d'une prédisposition à un trouble du poids dépendant de l'accumulation de graisses, comprenant :
l'obtention d'un échantillon prélevé chez un individu,
la détection du taux d'activité de la S6 kinase 1 dans ledit échantillon, et
la corrélation d'un changement dans l'activité de la S6 kinase 1 par rapport à une valeur ou une gamme de valeurs témoins normales avec une prédisposition à un trouble du poids dépendant de l'accumulation de graisses.

10. Méthode selon la revendication 9, **caractérisée en ce que** ledit changement est une augmentation dans l'activité de la S6 kinase 1 par rapport à une valeur ou une gamme de valeurs témoins normales et ledit trouble du poids est une surcharge pondérale ou l'obésité.
